# EUROPEAN PATENT APPLICATION

(11) **EP 2 060 559 A1**
(43) Date of publication of application: **20.05.2009**
(21) Application number: 08253756.4
(22) Date of filing: 19.11.2008
(51) Int. Cl.: C07C 69/83, C07C 215/30, C07C 217/48, C07D 333/16, C07D 333/20

(54) **Process for the preparation of enantiomerically pure 3-hydroxy-3-arylpropylamines and their optical stereoisomers**

(30) Priority: 19.11.2007 IN MU22802007
(71) Applicant: Cadila Pharmaceuticals Limited, Bhat, Ahmedabad 382 210 (IN)
(72) Inventor: Modi, Indravadan, Ambalal, Ahmedabad 382210 (IN); Shukla, Manish, Chandrakant, Ahmedabad 382210 (IN); Kagathara, Nirav, Keshavlal, Ahmedabad 382210 (IN); Jain, Pratima, Ahmedabad 382210 (IN); Korikana, Siva, Prasad, Ahmedabad 382210 (IN); Tarannum, Ahmedabad 382210 (IN); Ponnaiah, Ravi, Ahmedabad 382210 (IN); Khamar, Bakulesh, Mafatlal, Ahmedabad 382210 (IN)
(74) Representative: Towler, Philip Dean

(57) **Abstract**

The invention provides a process for the preparation of enantiomerically pure 3-hydroxy-3-arylpropylamines via novel semiester derivatives containing o-carboxy benzoyl group as intermediates. The 3-hydroxy-3-arylpropylamines serve as precursor for the preparation of serotonin or noradrenalin reuptake inhibitors such as duloxetine, atomoxetine, fluoxetine, nisoxetine and norfluoxetine.

## Description

### FIELD OF THE INVENTION:

The present invention relates to a process for the preparation of enantiomerically pure 3-hydroxy-3-arylpropylamines via novel semiester derivatives containing o-carboxy benzoyl group as intermediates. The 3-hydroxy-3-arylpropylamines serve as precursor for the preparation of serotonin or noradrenalin reuptake inhibitors such as duloxetine, atomoxetine, fluoxetine, nisoxetine and norfluoxetine.

### BACKGROUND OF THE INVENTION:

The enantiomerically pure 3-hydroxy-3-arylpropylamines of formula-I 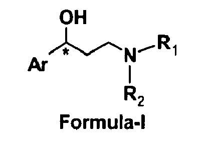 and its pharmaceutically acceptable salts, are valuable intermediates for the synthesis of pharmaceutical active substances such as e.g. (R)-atomoxetine, (S)-fluoxetine, (S)-duloxetine or (S)-norfluoxetine, which belong to the norepinephrine and serotonin reuptake inhibitors used pharmaceutically as antidepressants or agents for treating urinary incontinence. In these compounds of formula-1, Ar is an aryl group, of which phenyl and 2-thienyl are preferred; R₁, R₂ individually represent hydrogen or C₁-C₁₀ alkyl, and benzyl groups, the carbon center marked with "*" can be racemic or enantiomerically enriched (R) - or (S) - configuration.

Various processes are disclosed in the prior art for producing enantiomerically pure 3-hydroxy-3-arylpropylamines.

Y. Gao and K. B. Sharpless in "J. Org. Chem., 53, 4081 (1988)" disclose a process in which cinnamyl alcohol is subjected to Sharpless Oxidation to give optically active 2,3-epoxycinnamyl alcohol, which is reduced with Red-Al to give the 1,3-diol followed by mesylation at 1-position and reacted with an amine.

E. J. Corey and G. A. Reichard in "Tetrahedron Lett., 30, 5207 (1989)" disclose a process in which 3-chloropropiophenone is subjected to borane reduction in the presence of an optically active oxazaborolidine to give optically active 3-chloro-1-phenylpropanol, which is subsequently treated with sodium iodide and then reacted with an amine.

S. Sakuraba and K. Achiwa, in "Synlett, 689 (1991)" disclose a process in which optically active amino alcohol obtained by asymmetric hydrogenation of a β-amino ketone hydrochloride with MCCPM-Rh catalyst.

D. Mitchell and T. M. Koenig in "Synthetic Communications, 25, 1231 (1995)" disclose a process in which an optically active β-cyan alcohol is obtained by a reaction between optically active styrene oxide and acetone cyanohydrin, and the cyano group is then reduced.

T. M. Koenig and D. Mitchell in "Tetrahedron Lett., 35, 1339 (1994)" disclose a process in which a β-cyan alcohol racemate is synthesized by a reaction between benzaldehyde and acetonitrile, and the cyano group is further reduced to give a γ-amino alcohol, which is then optically resolved to give an optically active γ-amino alcohol.

J. R. Dehli and V. Gotor in "Tetrahedron: Asymmetry, 11, 3693 (2000)" disclose a process for producing an optically active β-cyano alcohol by asymmetric reduction of benzoylacetonitrile, which is an α-cyano ketone, there is a process in which the asymmetric reduction is carried out by a microorganism. This process has the defects that the reaction yield is low and the absolute configuration of the alcohol so obtained is limited to a specific type.

Production of an optically active amino alcohol from a cyano ketone using a metal catalyst, a borane reduction process (using an optically active oxazaborolidine as a catalyst) is disclosed in WO 00/07976, but this process has the problem of borane liquid waste since the borane compound and the cyano ketone are used in equimolar amounts.

Various techniques have been proposed for reducing a ketone to an alcohol using a transition metal complex. JP, A, 8-225466 discloses a hydrogen reduction process using an optically active phosphine and an optically active amine as ligands of a transition metal complex, and JP, A, 11-189600 relates to a process for producing an optically active alcohol from a carbonyl compound using a novel ruthenium complex having phosphine and amine ligands etc.

Furthermore, JP. A, 9-157196 and JP. A, 11-322649 disclose reactions of transition metal complexes having as a ligand an optically active nitrogen-containing compound using a hydrogen donor compound instead of hydrogen. JP, A, 9-157196 illustrates as reaction substrates, a large number of carbonyl compounds having as one or more substituents; an aromatic compound, a heterocyclic compound, an aliphatic compound, etc., and although they include carbonyl compounds substituted with a cyano group, which is electron-withdrawing and shows strong coordination to a transition metal, or a nitro group, which is electron-withdrawing, in the embodiments there is no mention of a reaction for their reduction into a cyano alcohol or a nitro alcohol using as a substrate a cyano ketone or a nitro ketone having a cyano or nitro group on the α-carbon, and neither is there mention of other cyano ketones and nitro ketones.

Different other conventional processes for producing optically active amino alcohols are: (1) a process in which an α-azido ketone is asymmetrically reduced using bread yeast and the azido group of the β-azido alcohol thus obtained is reduced (J. S. Yadav, P. T. Reddy, S. Nanda, and A. B. Rao, Tetrahedron: Asymmetry, 12, 63 (2001)), (2) a process in which an β-amido ketone is asymmetrically reduced using a metal catalyst to give a β-amido alcohol, and a protecting group on the nitrogen is removed (A. Kawamoto and M. Wills, Tetrahedron: Asymmetry, 11, 3257(2000)), and (3) a process in which an β-amino ketone is asymmetrically reduced using a metal catalyst (Comprehensive Asymmetric Catalysis, I. Springer, p. 210-212 (1999)).

Among the processes described above, process (1) has the defects that the type of reaction substrate is restricted moreover; the absolute configuration of the alcohol so obtained is limited to a specific type. In process (2), the yield is low when removing the protecting group on the nitrogen, which is not suitable for industrial production. Process (3) cannot always be said to have generality since, the type of a suitable substrate depends on the type of a metal catalyst. Furthermore, the type of substituent on the nitrogen is restricted, and it lacks versatility.

The pure enantiomer of the 3-hydroxy-3-phenyl-propylamine is a valuable intermediate for the synthesis of pharmaceutical active substances such as e:g. (R)-atomoxetine, (S)-fluoxetine or (S)-norfluoxetine. Numerous alternative methods of producing enantiomerically pure phenylpropylamines are disclosed in prior art.

Sharpless et al. propose enantioselective epoxidation (J. Org. Chem. 1988, 53, 4081) for preparing enantiomerically pure compounds of this type.

Corey et al. propose enantioselective oxazaborolidine-catalysed ketone reduction (Tetrahedron Lett. 1989, 30 5207) for preparing enantiomerically pure compounds of this type.

Koenig et al. indicate a conventional chemical method for preparing a racemic phenylpropylamine with subsequent racemate cleavage using chiral mandelic acid (Tetrahedron Lett. 1994, 35, 1339).

T. Ohkuma et al. propose in Organic Letters 2000, Vol. 2 No. 12 1749-1751 the enantioselective hydrogenation of 3-dimethylamino-1-(2-thienyl)-propanone using a chiral ruthenium catalyst in the presence of potassium tert-butoxide.

The pure enantiomer of the 3-hydroxy-3-(2-thienyl)-propylamine is valuable intermediate product for the synthesis of (S)-duloxetine. Numerous alternative methods of producing enantiomerically pure thienylpropylamines are disclosed in prior art.

A process for preparing enantiomerically enriched (1S)-3-(methylamino)-1-(2-thiophen- yl)-1-propanol starting from 1-(thiophen-2-yl)-3-chloropropan-1-one is described in Chirality 2000, 12, 26-29. After the reduction to racemic 3-chloro-1-(2-thienyl)-1-propanol, the racemate is enzymatically separated and the (S)-enantiomer is further reacted with Nal and methylamine to give (S)-3-(methylamino)-1-(thiophen-2-yl)-propan-1-ol. This method has the disadvantage that enzymatic racemate separations can in principle only provide 50% of the desired enantiomer and the overall yield is therefore economically unacceptable. A similar synthetic route is described in J. Lab. Comp. Radiopharm. 1995, 36, 213-223, in which 1-(2-thienyl)-3-chloropropan-1-one is asymmetrically reduced with borane and an oxazaborolidine. The yield in this step is only 61 %, which makes the overall process uneconomic.

Enantiomeric purity is important in the field of pharmaceuticals, where many of the prescribed drugs exhibit chirality, and the two isomers exhibit different potency (Drugs news & perspective, 1997, vol.3, p139-160). Furthermore, enantiomeric purity is important since certain isomers may actually be deleterious rather than simply inert. Therefore there is a need to obtain the desired enantiomer in high enantiomeric purity.

There is a long-felt need for an efficient and commercially feasible process for the preparation of enantiomerically pure 3-hydroxy-3-arylpropylamines.

### SUMMARY OF THE INVENTION:

The object of present invention is to provide a cost effective process for the production of enantiomerically pure 3-hydroxy-3-arylpropylamines having the formula-1, 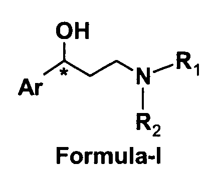 wherein Ar, R₁ & R₂ are as defined above, in high yield, high optical purity on a commercial scale.

Another object of the invention is to provide a process for converting racemic 3-halo-1-aryl-1-propanol to chiral 3-substitued amino-1-aryl-1-propanol.

Another object of the invention is to provide a process for preparing compound of formula-I, by reacting an enantiomerically pure compound of formula-V with ammonia, primary or secondary amines, to give a compound of formula-I

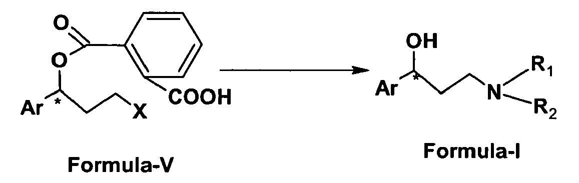

Yet another object of the invention is to provide a process by which R-atomoxetine, S-fluoxetine, S-duloxetine, and S-norfluoxetine and nisoxetine may be prepared in a high optical and chemical purity.

Yet another object of the invention encompasses 2-[(3-chloro-1-arylpropoxy) carbonyl] benzoic acid of the following structure 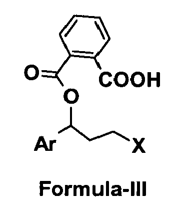 wherein Ar, X are defined above.

Yet another object of the invention encompasses a chiral amine salt of 2-[(3-chloro-1-arylpropoxy) carbonyl] benzoic acid of the following structure

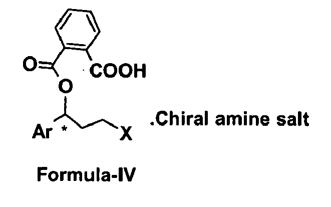

In yet another embodiment, the invention encompasses (R) or (S) - 2-[(3-chloro-1-arylpropoxy) carbonyl] benzoic acid of the following structure [ Formula-V]

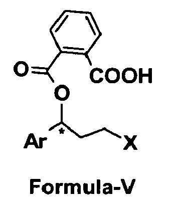

### DETAILED DESCRIPTION OF THE INVENTION:

The present invention provides a process for the preparation of enantiomerically pure 3-hydroxy-3-arylpropylamines having the formula-I 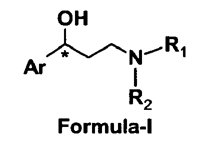 wherein Ar is an aryl group, of which phenyl and 2-thienyl are preferred; R₁, R₂ individually represent hydrogen or C₁-C₁₀ alkyl, and benzyl groups, the carbon center marked with "*" can be either a racemic or enantiomerically enriched (R) - or (S) - configuration, and its pharmaceutically acceptable salts. The present preparation is carried out according to following scheme. 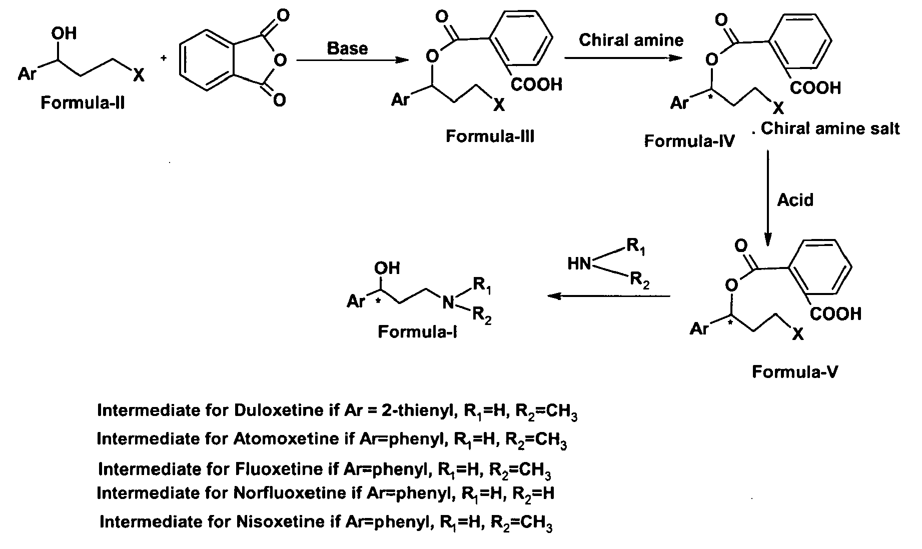
(i) acylating the racemic alcohol of the formula II with phthalic anhydride in the presence of a base to give the racemic phthalic semiester of the formula III;
(ii) resoluting phthalic semiester of the formula III, by means of chiral amine to give the corresponding salt of formula IV;
(iii) isolating enantiomerically pure phthalic semiester of the formula V;
(iv) reacting the enantiomerically pure phthalic semiester of the formula V with amine HNR₁R₂ (optionally using iodide exchange catalysis and/or phase transfer catalysis) to give the enantiomerically pure 3-hydroxy-3-arylpropylamines of the formula-I.

This process comprises the steps:
(i) The racemic alcohol of the formula-II is acylated with phthalic anhydride in the presence of a base to give the racemic phthalic semiester of the formula III. 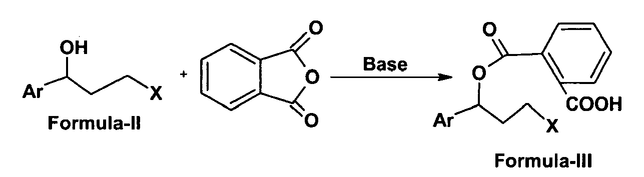 The starting alcohol II is generated, in racemic form, by reducing the corresponding ketone 3- [3-halo-1-aryl-1-propanone], with a reducing agent, such as, for example, sodium borohydride.
   In step (i), the base is selected from tertiary amines such as pyridine, triethylamine, and 4-DMAP. Preferably the organic base is pyridine or 4-DMAP. The amount of base used in the reaction is about 0.25 to about 1 mole per mole of 3-halo-1-aryl-propanol. Preferably, base is added in an amount of 0.54 moles per mole of 3-halo-1-aryl-propanol. The reaction is carried out at 50-70°C, preferably at 60-65° C. After completion of the reaction, pH of the reaction mass is adjusted to 2-3 by addition of aqueous HCl. The mixture is extracted with organic solvent, such as o-xylene, toluene, ethylacetate etc. and washed with water and then with brine solution. The solvent is distilled out and the residue is treated with aliphatic alkanes like n-hexane, cyclohexane, preferably cyclohexane, to give the compound of Formula -III.
(ii) Phthalic semiester of the formula III is resolved by means of chiral amine to give the corresponding salt formula IV. 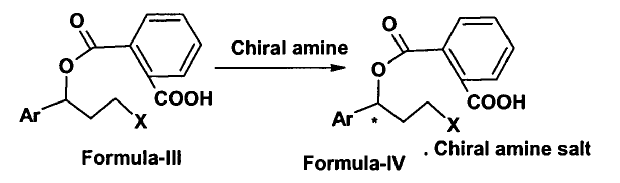 In step (ii), resolution is carried out via chiral amine. Depending on which enantiomer of the alcohol I is required, chiral amine (S- or R-isomer) is selected. Preferably 1-phenyl ethyl amine (S- or R-isomer) is used. The solvent used for the reaction may be selected from alcohols, ethers and aromatic hydrocarbons such as toluene, xylene, preferably toluene. The chiral amine salt is further crystallized from alcohols, such as methanol, ethanol, isopropyl alcohol, ethers such as diisopropyl ether and aromatic hydrocarbon such as toluene, or mixtures thereof.
(iii) Enantiomerically pure phthalic semiester of the formula-V is isolated. 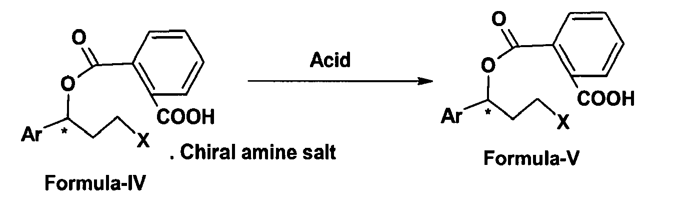 In step (iii), compound of formula-IV is converted to compound of formula-V. Acid is used to isolate the salt of phthalic semiester-IV into the enantiomer of the phthalic semiester of alcohol V. Here acid is an organic acid, or alcoholic HCl or aqueous HCl. Organic solvent used for the extraction is chlorinated solvent such as dichloromethane. The anti-solvent used for isolation is selected from hexane, heptane or cyclohexane.
(iv) Enantiomerically pure phthalic semiester of the formula-V is reacted with amine HNR₁R₂ (optionally using iodide exchange catalysis and/or phase transfer catalysis) to give the enantiomerically pure 3-hydroxy-3-arylpropylamine of the formula-I.

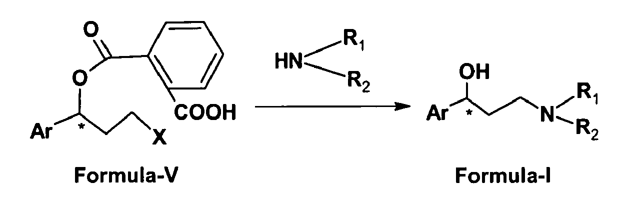

In step (iv), the desired enantiomerically pure phthalic semiester is reacted with amine of formula NH(R₁)(R₂) (optionally using iodide exchange catalysis and/or phase transfer catalysis) to give the enantiomerically pure 3-hydroxy-3-arylpropylamine (formula-I). Here amine is selected from ammonia, primary amine or secondary amine as per the requirement of the final drug. To enhance the rate of reaction of amine with halide, an iodide ion source such as Nal or KI is optionally used in conjunction with a phase transfer catalyst. The phase transfer catalyst may be, but is not limited to, a quaternary ammonium salt, preferably tetrabutyl ammonium bromide [TBAB]. The solvent for the reaction is C₁-C₇ alcoholic solvent, preferably methanol. The organic solvent used for extraction is typically a chlorinated solvent such as dichloromethane, dichloroethane, chloroform etc.

Synthesis of duloxetine, fluoxetine, atomoxetine nisoxetine, norfluoxetine or a derivative thereof using an enantiomerically pure 3-hydroxy-3-arylpropylamine formed above as a starting material can be carried out by a conventionally known synthetic process, for example, according to processes described in US Pat. No. 5362886 (for Duloxetine), US Pat. No.6541668 (for atomoxetine), Mitchell et al. (Mitchell, D. and Koenig, T. M., Synthetic Communications, 25(8), 1231-1238 (1995)) and Koenig et al. (Koenig, T. M. and Mitchell, D., Tetrahedron Letters, 35(9), 1339-1342 (1994)).

The present invention is illustrated by following non-limiting examples.

### Example-1

### Preparation of 2-[(3-chloro-1-thien-2-ylpropoxy) carbonyl] benzoic acid

A mixture of 3-chloro-1-(2-thienyl)-propan-1-ol (340 g, 1.92 mol), phthalic anhydride (313.6 gm, 2.12 mol), pyridine (85 ml, 1.07mol) was stirred at 60-65°C for 4 hours. The reaction mixture was cooled to room temperature, diluted with o-xylene (1700 ml), and then dil HCl (67.15 ml in 167.8 ml water) was added. The reaction mass was stirred for 15-20 minutes. Then water was added to the reaction mass at room temperature and stirred for 15 minutes. The layers were separated and the organic layer was washed with water. Two third volume of organic layer was evaporated under high vacuum at 45-50°C. Solid material was precipitated. Cyclohexane (1020 ml) was added into it and stirred well. The solid was filtered and washed with cyclohexane.
Yield: 493 g. (79 %)
¹H NMR (CDCl₃, 300 MHz): δ =6.76-7.92 (m, 7H, Ar-H), 6.51-6.55 (m, 1H, -CH), 3.50-3.69 (m, 2H, -CH2), 2.36-2.67 (m, 2H, -CH2) ppm.
M/Z M⁺ 325.

### Example-2

### Preparation of (1S)-(-)-1-phenylethanamine salt of 2-[(3-chloro-1-thien-2-ylpropoxy) carbonyl] benzoic acid

A mixture of 2-[(3-chloro-1-thien-2-ylpropoxy) carbonyl] benzoic acid (200 g, 0.062 mol), toluene (1600 ml), S-(-)-α-methyl benzyl amine (74.57 g, 0.062 mol) was stirred at 60-65°C for 1 hr. The reaction mixture was gradually cooled to room temperature. The solid mass separated. The reaction mass was filtered and washed with toluene to give a crude salt. The crude salt was crystallized from toluene : methanol (7:1) to give pure resolved salt of 2-[(3-chloro-1-thien-2-ylpropoxy) carbonyl] benzoic acid.
Chiral purity: > 99.90 %
¹H NMR (CDCl₃, 300 MHz): δ =7.56-7.59 (m, 1 H, -COOH), 6.89-7.35 (m, 12H, Ar-H), 6.3-6.35 (m, 1H, -CH), 4.18-4.25 (m, 1H, -CH (NH2, CH3, Ph)), 3.4-3.6 (m, 2H, - CH2), 2.24-2.31 & 2.46-2.55 (m, 2H, -CH2), 1.43-1.46(doublet, 3H, -CH3) ppm.
M/Z M⁺ 446.

### Example-3

### Preparation of 2-[((1S)-(-)-3-chloro-1-thien-2-ylpropoxy) carbonyl] benzoic acid

A mixture of resolved half ester salt (109 g), IPA (327 ml) was stirred at room temperature for 15 minutes. The pH of the solution was adjusted ∼ 4 - 5 by addition of HCl solution (24 ml in 50 ml water). The mixture was stirred at room temperature till a clear solution was obtained. Then water (900 ml) was added into the reaction mass. The mixture was then extracted with methylene chloride (545 ml) and the extract was washed with water. The organic layer was dried over sodium sulphate. The organic layer was distilled under high vacuum at 40°C and traces of methylene chloride was removed using cyclohexane. Cyclohexane was added, the solid was filtered and washed with cyclohexane and dried.
Yield: 70 g (89 %)
Chiral purity: >99.94 %
SOR: - -31.37° (c=1 % methanol)
¹H NMR (CDCl₃, 300 MHz): δ =6.76-7.92 (m, 7H, Ar-H), 6.51-6.55 (m, 1 H, -CH), 3.50-3.69 (m, 2H, -CH2), 2.36-2.67 (m, 2H, -CH2) ppm.
M/Z M⁺ 325.

### Example-4

### Preparation of (1S)-(-)-3-(methylamino)-1-thien-2-ylpropan-1-ol

A mixture of resolved half ester (110.0 g, 0.338 mol), methanol (550 ml, 5V), 40 % monomethyl amine (1100 ml, 10V), sodium iodide (50.8 g, 0.338 mol) and TBAB (10.0 gm, 0.0338mol) was stirred at 60-65°C for 6-8 hours. The reaction mixture was cooled to room temperature. Two third volume of reaction mass was distilled under vacuum at 40°C. Toluene (150 ml) was added and the reaction mass was stirred for 10-15 minutes. 30% NaOH (150 ml) solution was added. This operation was repeated 3 times and the layers were separated. All the toluene layers were combined and distilled under reduced pressure. Cyclohexane was added to it and the mass was stirred for 30 min. The solid was filtered and washed with cyclohexane.
Yield: 35 g
Chiral purity: >99.94 %
SOR: - -16.5° (c=1% methanol)

### Example-5

### Preparation of S-(+)-Duloxetine hydrochloride

A mixture of sodium hydride (2.08 g, 60 %, 0.0578 mol) and DMA (27 ml) was stirred at 60° C. (1S)-(-)-3-(methylamino)-1-thien-2-ylpropan-1-ol (9.0 g in 9.0 ml DMA, 0.052 mol) was added and stirred for 30 mins. 1-Fluoro naphthalene (7.59 g of in 9.0 ml DMA, 0.052 mol) was added and the reaction mass was stirred at 75-80° C for 8-10 hrs. The reaction mixture was cooled to room temperature and poured into water (250 ml). The mixture was extracted with ethyl acetate (150 ml x 2) and extract was washed with water. The ethyl acetate layer was evaporated to dryness and the residue was dissolved in ethyl acetate (20 ml) and oxalic acid dihydrate (4.77 gm in 75 ml ethyl acetate) was added and the mixture was stirred at room temperature for 30 mins. The resulting suspension was filtered and washed with ethyl acetate to give duloxetine oxalate.
Wt. of dry material: 12.5 g.
The solid was converted to free base with sodium hydroxide solution and extracted with ethyl acetate. The extracts were washed with water and then evaporated to give Duloxetine base.
Wt. of oil 6.0 g.
The duloxetine base (6.0 g) was dissolved in ethyl acetate (48 ml). Ethyl acetate HCl/IPA:HCl solution (2-7 %) was added and stirred for 30 mins. The solid was filtered and washed with ethyl acetate (25 ml). The material was dried under vacuum to give Duloxetine HCl.
Yield: - 6 g
Chiral purity: >99.90 %

### Example-6

### Preparation of 2-[(3-chloro-1-phenylpropoxy) carbonyl] benzoic acid

A mixture of 3-chloro-1-phenylpropan-1-ol (330 g, 1.93 mol), phthalic anhydride (286.45 g, 1.93 mol) and pyridine (82.5 ml, 1.02 mol) was stirred at 55-60°C for 2.5 to 3 hrs. The reaction mass was diluted with ethyl acetate (660 ml) and added aqueous HCl (1100ml, 137.58 con. HCl in 962ml water) at 55-60°C. The reaction mass was stirred for 15 minutes. Cyclohexane (1.65 liter) was added and reaction mass was stirred at 60-65°C for half an hour. The layers were separated at 60-65°C. The organic layer was washed with water (825 ml x 3) at 60-65°C. The organic layer was cooled to 10-15°C, filtered and washed with cyclohexane (370 ml) and dried to give white solid.
Wt. of solid: 509 g
¹H NMR (CDCl₃, 300 MHz): δ =7.25-7.91 (m, 9H, Ar-H), 6.17-6.21 (m, 1H, O-CH), 3.59-3.65 (m, 1H, -CH-Cl), 3.44-3.52 (m, 1H, -CH-Cl), 2.48-2.59 (m, 1H, -CH-CH2-Cl) 2.22-2.33 (m, 1H, -CH-CH2-Cl) ppm.
M/Z M⁻¹ 317.1.

### Example-7

### Preparation of (1R)-(+)-1-phenylethanamine salt of 2-[(3-chloro-1-phenylpropoxy) carbonyl] benzoic acid

2-[(3-chloro-1-phenylpropoxy) carbonyl] benzoic acid (120 g, 0.377 mol) , toluene (960 ml), and R-(+)-α-methyl benzyl amine (45.73 ml, 0.36mol) were stirred at room temperature for 30 minutes. Temperature was raised to 70-75°C and stirred till clear solution was obtained. The reaction mass was cooled to room temperature, stirred for half an hour, filtered and washed with toluene. The obtained product was crystallized from toluene:isopropanol (8:2) mixture.
Wt. of solid: - 70 g
Chiral purity: - >99.97%
¹H NMR (CDCl₃, 300 MHz): δ =7.19-7.65 (m, 14H, Ar-H), 6.02-6.05 (m, 1H, O-CH), 5.5-5.6(s, 2H, -NH2), 4.1-4.13 (quartet, 1H, CH3-CH), 3.55-3.59 (m, 1H, -CH-Cl), 3.43-3.55 (m, 1H, -CH-Cl), 2.35-2.48 (m, 2H, -CH-CH2-Cl), 1.36-1.38 (doublet, 3H, -CH-CH3) ppm.
M/Z M⁺ 440.2

### Example-8

### Preparation of 2-({[(1R)-(-)-3-chloro-1-phenylpropyl] oxy} carbonyl) benzoic acid

A mixture of resolved half ester salt (300 g, 0.68mol) and IPA (1.2 liter) was stirred at room temperature for 15 minutes. The pH of the solution was adjusted - 4-5 by addition of aqueous HCl solution (150.8ml con. HCl in 301.6 ml water) at room temperature. The mixture was stirred at room temperature till a clear solution was obtained. Reaction mass was extracted with MDC (750ml x 2). The organic layer was washed with water (1.5 liter x 3), dried over sodium sulphate. The solvent was distilled off under reduced pressure to obtain yellow oil. Cyclohexane (600 ml) was added and stirred at 10-15°C. Filtered and washed with cyclohexane (150ml).
Wt. of solid: 202g
Chiral purity: >99.95%
SOR: -10.72° (c=1% in methanol)
¹H NMR (CDCl₃, 300 MHz): δ =7.25-7.91 (m, 9H, Ar-H), 6.17-6.21 (m, 1H, O-CH), 3.59-3.65 (m, 1H, -CH-Cl), 3.44-3.52 (m, 1H, -CH-Cl), 2.48-2.59 (m, 1H, -CH-CH2-Cl) 2.22-2.33 (m, 1H, -CH-CH2-Cl) ppm.
M/Z M⁻¹ 317.1.

### Example-9

### (1R)-(+)-3-(methylamino)-1-phenylpropan-1-ol

A mixture of 2-({[(1R)-(-)-3-chloro-1-phenylpropyl]oxy} carbonyl) benzoic acid (140 g, 0.44 mol), methanol (700 ml), Nal (6.58 g, 0.044 mol) was stirred for 30 minutes at 50-55°C. Aqueous monomethyl amine (1.4 liter) was added and the reaction mixture was stirred at 50-55°C for 30 minutes. Tetrabutyl ammonium bromide [TBAB] (1.415 g, 0.0044mol) was added and stirred at 60-65°C for 9-10 hours. The water and methanol were distilled at 45-50°C under vacuum and toluene (280 ml) was added. NaOH solution (30%) was added. The toluene layer was separated, this process was repeated twice and all the toluene layers were collected. The combined toluene extract was washed with brine solution (250ml x 2) and dried over anhydrous Na₂SO₄ (300 g). The organic layer was distilled under high vacuum to give pale yellow oil which was further treated with hexane to give solid product. Wt. of product = 30 g.
Chiral purity: >99.98 %
SOR= +38.36° (c=1 % methanol)

### Example-10

### Preparation of R-(-)-Atomoxetine hydrochloride

A mixture of sodium hydride (0.8 g, 60 %, 0.02 mol), DMSO (9 ml), and (1R)-(+)-3-(methylamino)-1-phenylpropan-1-ol (3.0 g in 3.0 ml DMSO, 0.018 mol) was stirred for 15 min. Potassium benzoate (3.2 g, 0.02 mol) was added and stirred for 1 hr at 50-55°C. o-fluoro toluene (5.99 g in 3.0 ml DMSO, 0.054mol) was added and the reaction mass was stirred at 50-55°C for 15 minutes. The reaction mass was heated to 110-120°C for 6-7 hrs. The reaction mass was cooled and poured into 90 ml of water. Toluene (30 ml) was added and the layers were separated. The aqueous layer was further extracted with toluene (15 ml x 2).The organic layers were combined, and washed with water (30 ml) and dried over anhydrous sodium sulfate. The organic layer was distilled to give an oil.
Wt. of the oil= 4.0 g.
The oil (4.0 g) was dissolved in toluene (32 ml). Oxalic acid dihydrate (2.0 g) in isopropyl alcohol (10 ml) was added and stirred for 1 hr at temperature 25-30°C.The precipitated solid was filtered and washed with toluene (8ml x 2) to give atomoxetine oxalate.
Wt. of dry material: 3.64 g.

Atomoxetine oxalate was stirred (2.5 g) with ethyl acetate (25 ml) and NaOH solution (0.61 gm in 25 ml water) was added. The layers were separated. Aq. Layer was further extracted with ethylacetate (12.5 ml x 2). Ethyl acetate layers were combined and washed with water and dried over anhydrous sodium sulfate and distilled to give atomoxetine base as an oil.
The atomoxetine base (1.7 g) was dissolved in ethyl acetate (13.6 ml). Ethyl acetate HCl solution (4.9 ml 5 %) was added. The reaction mixture was stirred for 1 hr, the precipitated solid was filtered and washed with ethyl acetate (10 ml). The wet cake was dried under vacuum to give atomoxetine HCl.
Yield: - 1.14 gm
Chiral purity: >99.90 %

## Claims

1. A process for the preparation of enantiomerically pure 3-hydroxy-3-arylpropylamines of formula I, comprising the steps of:
a) reacting compound of formula-II, 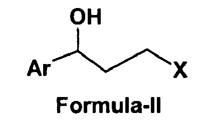 wherein Ar is an aryl group and X is halogen, with phthalic anhydride in the presence of a base and isolating the phthalic semiester of formula-III; 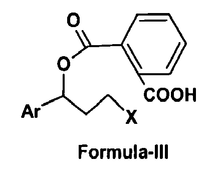
b) treating the compound of formula-III with chiral amine to produce corresponding salt of the formula-IV: 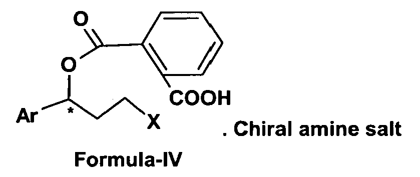
c) treating the compound of formula-IV with an acid to get the corresponding enantiomerically pure phthalic semiester of the formula-V; 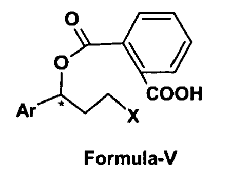
d) reacting the enantiomerically pure phthalic semiester of the formula-V with amine HNR₁R₂ (optionally using iodide exchange catalysis and/or phase transfer catalysis) to give the enantiomerically pure 3-hydroxy-3-arylpropylamines of the formula-I.

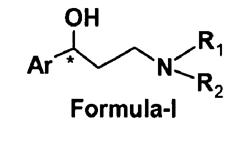

2. A process as claimed in claim 1, wherein Ar is phenyl or 2-thienyl.

3. A process as claimed in claim 1, wherein X is selected from halogen group.

4. A process as claimed in claim 1, wherein base used in step (a), is selected from tertiary amine such as pyridine, triethylamine and 4-DMAP (4-Dimethylaminopyridine) preferably pyridine or 4-DMAP.

5. A process as claimed in claim 1, wherein chiral amine used in step(b), is enantiomerically pure chiral amine such as S-(+)-1-phenylethyl amine or R-(-)-1-phenytethyl amine .

6. A process as claimed in claim 1, wherein acid is used in step (c ), is an organic acid or a mineral acid, preferably hydrochloric acid.

7. A process as claimed in claim 1, wherein iodide compound used in step-(d) is sodium iodide or potassium iodide, preferably potassium iodide.

8. A process as claimed in claim 1, wherein the phase transfer catalyst used in step-(d) is selected from quaternary ammonium salts preferably benzyl triethyl ammoniun chloride,TBAB and their like, more preferably TBAB.

9. A process as claimed in claim 1, wherein the compound I is N-methyl-3-hydroxy-3-phenylpropylamine and its (R) and (S)-enantiomers.

10. A process as claimed in claim 1, wherein the compound of formula-I is N-methyl-3-hydroxy-3-(2-thienyl)propylamine and its (R) and (S)-enantiomers.

11. A compound of the formula-III, 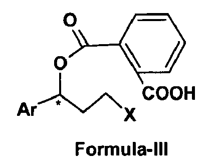 wherein Ar denotes aryl or substituted aryl and X denotes halogen.

12. A compound of the formula-IV, 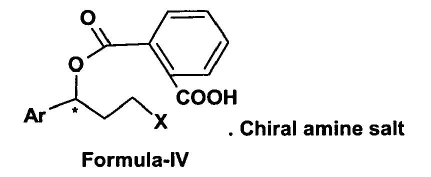 wherein Ar denotes aryl or substituted aryl and X denotes halogen.

13. A process for the preparation of enantiomerically pure 3-hydroxy-3-(2-thienyl) propylamine of formula-Ia 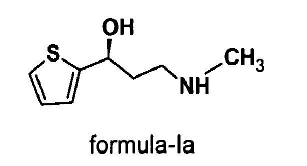 comprising the steps of:
a) reacting compound of formula-la, 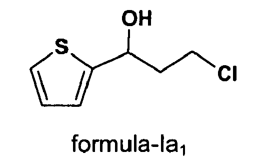 with phthalic anhydride in the presence of a base and isolating the phthalic semiester of formula -Ia_{2;} 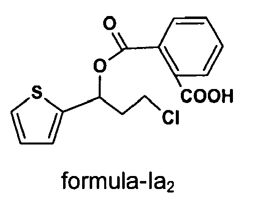
b) treating the phthalic semiester obtained in step (a) with S-(-)-α-methyl benzyl amine to produce corresponding salt of the formula - Ia₃; 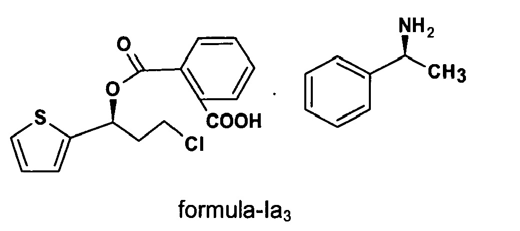
c) treating the salt obtained in step (b) with acid to get the corresponding enantiomerically pure phthalic semiester of the formula-la₄ ; 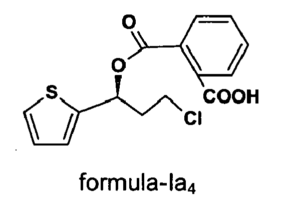
d) reacting the enantiomerically pure phthalic semiester obtained in step (c) with amine HNR₁R₂ (optionally using iodide exchange catalysis and/or phase transfer catalysis) to give the enantiomerically pure 3-hydroxy-3-(2-thienyl) propylamine of the formula-Ia.

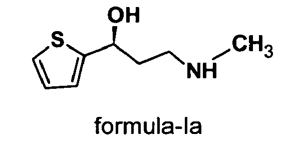

14. A process for the preparation of enantiomerically pure 3-hydroxy-3-phenylpropylamine of formula-Ib 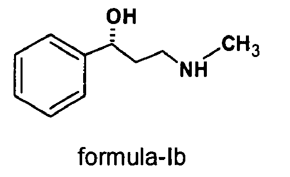 comprising the steps of:
a) reacting compound of formula-I_{b1} 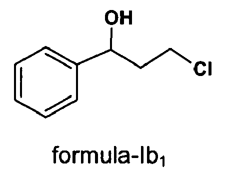 with phthalic anhydride in the presence of a base and isolating the phthalic semiester of formula-I_{b2;} 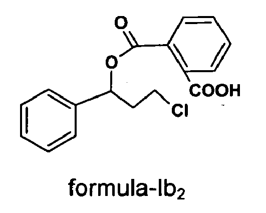
b) treating the phthalic semiester obtained in step (a) with R-(+)-α-methyl benzyl amine to produce corresponding salt of the formula-Ib_{3;} 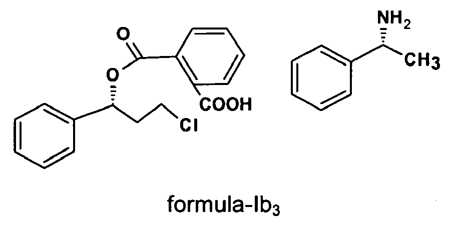
c) treating the salt obtained in step (b) with acid to get the corresponding enantiomerically pure phthalic semiester of the formula-Ib₄; 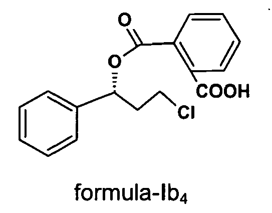
d) reacting the enantiomerically pure phthalic semiester obtained in step (c) with amine HNR₁R₂ (optionally using iodide exchange catalysis and/or phase transfer catalysis) to give the enantiomerically pure 3-hydroxy-3-phenylpropylamine of the formula-Ib.

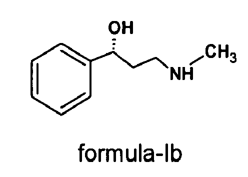

15. A compound of formula-Ia₄

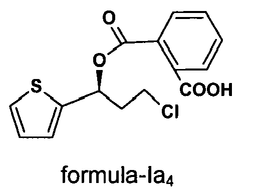

16. A compound of formula-Ib₄

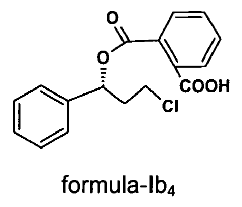

17. A enantiomerically pure compound of formula-Ia₄ having chiral purity greater than 99.80%.

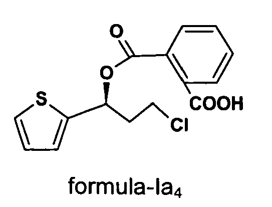

18. A enantiomerically pure compound of formula-Ib₄ having chiral purity greater than 99.80%.

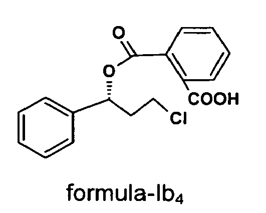

19. A process for preparing (R)-Atomoxetine hydrochloride comprising steps wherein :
a) 3-chloro-1-phenylpropan-1-of is treated with phthalic anhydride in the presence of a base to obtain 2-[(3-chloro-1-phenylpropoxy) carbonyl] benzoic acid;
b) 2-[(3-chloro-1-phenylpropoxy) carbonyl] benzoic acid is treated with (1R)-(+)-1-phenylethanamine to obtain 2-({[(1*R*)-(-)-3-chloro-1-phenylpropyl] oxy} carbonyl) benzoic acid salt of (1*R*)-1-phenylethanamine;
c) 2-({[(1R)-(-)-3-chloro-1-phenylpropyl] oxy} carbonyl) benzoic acid salt of (1*R*)-1-phenylethanamine is treated with acid to obtain 2-({[(1*R*)-(-)-3-chloro-1-phenylpropyl] oxy} carbonyl) benzoic acid;
d) 2-({[(1R)-(-)-3-chloro-1-phenylpropyl]oxy} carbonyl) benzoic acid is treated with methyl amine to obtain (1R)-(+)-3-(methylamino)-1-phenyl, 1-propanol;
e) (1R)-(+)-3-(methylamino)-1-phenyl, 1-propanol is treated with o-fluoro toluene in presence of sodium hydride and potassium benzoate in DMSO and finally converted to a hydrochloride salt.
